# EUROPEAN PATENT APPLICATION

(11) **EP 4 177 254 A1**
(43) Date of publication of application: **10.05.2023**
(21) Application number: 21833606.3
(22) Date of filing: 24.06.2021
(51) Int. Cl.: C07D 413/14, H05B 33/04, H05B 33/10, H01L 51/50

(54) **ORGANIC ELECTROLUMINESCENT ELEMENT**

(30) Priority: 03.07.2020 JP 2020115842
(71) Applicant: Hodogaya Chemical Co., Ltd., Tokyo, 105-0021 (JP)
(72) Inventor: CHIBA, Eriko, Tokyo 105-0021 (JP); KASE, Kouki, Tokyo 105-0021 (JP); YAMAMOTO, Takeshi, Tokyo 105-0021 (JP); HIRAYAMA, Yuta, Tokyo 105-0021 (JP); SURUGA, Kazuyuki, Tokyo 105-0021 (JP)
(74) Representative: Müller-Boré & Partner Patentanwälte PartG mbB
(86) International application number: PCT/JP2021/024002
(87) International publication number: WO 2022/004555

(57) **Abstract**

An object of the present invention is to provide an organic EL device having a capping layer constituted by a material having (1) high absorption coefficient, (2) high refractive index, (3) good thin film stability, (4) excellent durability, (5) excellent light resistance, (6) no absorption in the blue, green, and red wavelength regions, respectively.

Since an arylamine based material having a specific structure in the present invention is excellent in the stability and durability of the thin film, an organic EL device obtained by selecting amine compounds with high absorbance in the absorption spectrum at concentration 10⁻⁵ mol/L at wavelengths of 400 nm to 410 nm, from amine compounds having a specific benzazole ring structure with a high refractive index as a material of a capping layer, exhibits good characteristics.

## Description

### Technical Field

The present invention relates to compounds suitable for an organic electroluminescent device (hereinafter referred to as organic EL device), which is a preferred self-luminous device for various display devices, and more specifically relates to an amine compound having a benzazole ring structure and an organic EL device using the compound.

### Background Art

The organic EL device is a self-luminous device and has been actively studied for their brighter, superior visibility and the ability to display clearer images in comparison with liquid crystal devices.

In 1987, C.W. Tang and colleagues at Eastman Kodak developed a laminated structure device using materials assigned with different roles, realizing practical applications of an organic EL device with organic materials. These researchers laminated an electron-transporting phosphor and a hole-transporting organic substance and injected both charges into a phosphor layer to cause emission in order to obtain a high luminance of 1,000 cd/m² or more at a voltage of 10 V or less (see, for example, PTLs 1 and 2) .

To date, many improvements have been made for the practical application of organic EL devices. Various roles of the laminated structure have been further subdivided to provide a light emitting device that includes an anode, a hole injection layer, a hole transport layer, a light emitting layer, an electron transport layer, an electron injection layer, and a cathode sequentially on a substrate. In this light emitting device, efficiency and durability have come to be achieved by providing thereto a bottom emission structure that emits light from the bottom (see, for example, NPL 1).

Recently, a light emitting device with a top emission structure in which a metal having a high work function is used for an anode and light is emitted from the top is coming into use. In a light emitting device with a bottom-emission structure where light is taken out from the bottom having a pixel circuit, the area of the light emitting portion is limited, whereas in a light-emitting devices with a top-emission structure have the advantage that the light is taken out from the top and is not blocked by the pixel circuit, thus allowing a larger emission area. In the light emitting device with a top emission structure, translucent electrodes made of LiF/Al/Ag (see, for example, NPL 2), Ca/Mg (see, for example, NPL 3), LiF/MgAg, or the like are used for a cathode.

In such light emitting device, when light that is emitted by a light emitting layer and incident on another layer is incident at a certain angle or more, the light is totally reflected at an interface between the light emitting layer and the other layer. Thus, light that can be used has been limited to only a part of the emitted light. Recently, a light emitting device has been proposed in which a "capping layer" with a high refractive index is provided on the outside of a translucent electrode with a low refractive index, in order to improve the light extraction efficiency (see, for example, NPLs 2 and 3).

Regarding the effect of the capping layer in a light emitting device with a top emission structure, while a light emitting device using Ir(ppy)₃ as a light emitting material has a current efficiency of 38 cd/A in the case of not having a capping layer, the light emitting device has a current efficiency of 64 cd/A, in the case of using a ZnSe film with a thickness of 60 nm as a capping layer, which indicates that the efficiency is improved about 1.7 times. Furthermore, it is indicated that the maximum point of transmittance of the translucent electrode and capping layer does not necessarily coincide with the maximum point of efficiency, and that the maximum point of light extraction efficiency is determined by interference effects (see, for example, NPL 3) .

In the past, it has been proposed to use a metal mask with a high degree of definition to form the capping layer, but there is a problem that the metal mask is distorted by heat when used under high temperature conditions, resulting in a decrease in alignment accuracy. Therefore, ZnSe has a high melting point of 1100°C or higher (see, for example, NPL 3), and a high definition metal mask cannot be used to deposit ZnSe at precise positions, which may affect the light emitting devices themselves. Furthermore, even deposition by the sputtering method affects the light emitting devices, inorganic materials are not suitable for use as components of the capping layer.

In addition, when tris(8-hydroxyquinoline) aluminum (hereinafter abbreviated as Alq₃) is used as a capping layer to adjust the refractive index (see, for example, NPL 2), Alq₃ is known as an organic EL material commonly used as a green emitting material or electron transport material, it has a weak absorption around 450 nm, which is used for blue emitting materials, and has the problems of reducing the color purity and light extraction efficiency of blue emitting devices.

In addition, the devices fabricated with conventional capping layers allow light of 400 nm to 410 nm wavelength of sunlight to pass through, affecting the materials inside the devices and resulting in a decrease in color purity and light extraction efficiency.

In order to improve the device characteristics of organic EL device, especially to absorb light at a wavelength of 400 nm to 410 nm of sunlight, not to affect the materials inside the device, and to significantly improve the light extraction efficiency, materials with high absorption coefficient, high refractive index, and excellent thin film stability and durability are required as capping layer materials.

### Citation List

### Patent literatures

PTL 1: JP-A-8-048656
PTL 2: Japanese Patent No. 3194657
PTL 3: WO 2014/009310
PTL 4: WO 2013/038627

### Non-Patent Literatures

NPL 1: The Japan Society of Applied Physics, 9th Lecture Preprints, pp. 55-61 (2001)
NPL 2: Appl. Phys. Let., 78, 544 (2001)
NPL 3: Appl. Phys. Let., 82, 466 (2003)
NPL 4: J. Org. Chem., 71, 1802 (2006)
NPL 5: J. Org. Chem., 60, 7508 (1995)
NPL 6: Synth. Commun., 11, 513 (1981)
NPL 7: Appl. Phys. Lett., 98, 083302 (2011)

### Summary of the Invention

### Technical Problem

An object of the present invention is to improve the device characteristics of an organic EL device, in particular, not to affect the materials inside the device by absorbing light at a wavelength of 400 nm to 410 nm of sunlight, and to greatly improve light extraction efficiency. To this end, it is to provide an organic EL device having a capping layer containing a material having (1) high absorption coefficient at a wavelength of 400nm to 410 nm, (2) high refractive index, (3) good thin film stability, (4) excellent durability, (5) excellent light resistance, (6) no absorption in the blue, green, and red wavelength regions, respectively.

Physical properties of the material of the capping layer suitable for the present invention include (1) high absorption coefficient at a wavelength of 400 nm to 410 nm, (2) high refractive index, (3) vapor-deposit ability, (4) good thin film stability, and (5) high glass transition point. Physical properties of the organic EL device to be pursued in the present invention include (1) high absorption of light with a wavelength of 400 nm to 410 nm of sunlight, (2) high light extraction efficiency, (3) no occurrence of loss in color purity, (4) light transmission without changes over time, and (5) a long lifetime.

### Solution to Problem

For achieving the object, the present inventors have made earnest investigations, and focused on the fact that arylamine-based materials have excellent thin film stability and durability, and from an amine compound having a specific benzazole ring structure with a high refractive index, an amine compound having high absorbance in the absorption spectrum with a concentration of 10⁻⁵ mol/L at a wavelength of 400 nm to 410 nm was selected, and fabricated organic EL devices using the compounds as the materials constituting the capping layer of the devices, and thoroughly evaluated the properties of the devices, as a result of which the present invention has been accomplished.

Specifically, according to the present invention, the following organic EL devices are provided.
(1) An organic EL device in this order including at least an anode electrode, a hole transport layer, a light emitting layer, an electron transport layer, a cathode electrode, and a capping layer, the capping layer having a refractive index of 2.00 or more while light transmitted through the capping layer having a wavelength in a range of 400 nm or more and 500 nm or less, and a refractive index of 1.90 or more while light transmitted through the capping layer having a wavelength in a range of more than 500 nm and 570 nm or less, and the organic EL device containing an amine compound having a benzazole ring structure represented by the following general formula (1): 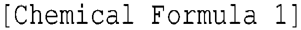 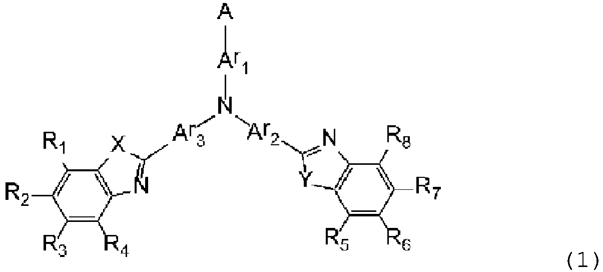
   wherein R₁ to R₈ may be the same or different, each represent a hydrogen atom, a deuterium atom, a fluorine atom, a chlorine atom, a cyano group, a nitro group, a linear or branched alkyl group having 1 to 6 carbon atoms that may have a substituent, a cycloalkyl group having 5 to 10 carbon atoms that may have a substituent, a linear or branched alkenyl group having 2 to 6 carbon atoms that may have a substituent, a linear or branched alkyloxy group having 1 to 6 carbon atoms that may have a substituent, a cycloalkyloxy group having 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or a substituted or unsubstituted aryloxy group, and these groups may be bonded to the benzene ring which bonded to, to form a ring through a single bond, a substituted or unsubstituted methylene group, an oxygen atom, or a sulfur atom, in which R₁ to R₄ or R₅ to R₈ bonded to the same benzene ring may be bonded to each other to form a ring through a single bond, a substituted or unsubstituted methylene group, an oxygen atom, or a sulfur atom; X and Y may be the same or different, and each represent an oxygen atom or a sulfur atom; Ar₁ to Ar₃ may be the same or different, and each represent a divalent group of a substituted or unsubstituted aromatic hydrocarbon, a divalent group of a substituted or unsubstituted aromatic heterocyclic ring, or a divalent group of a substituted or unsubstituted condensed polycyclic aromatic; and A represents a monovalent group represented by the following general formula (2) having a bonding site at any one of R₉ to R₁₅: 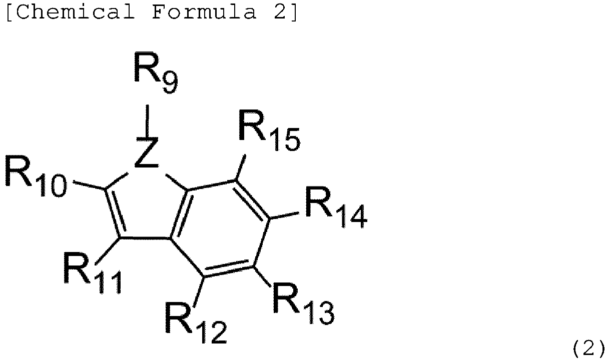
   wherein R₉ to R₁₅ may be the same or different, and each represent a linking group as a bonding site, a hydrogen atom, a deuterium atom, a fluorine atom, a chlorine atom, a cyano group, a nitro group, a linear or branched alkyl group having 1 to 6 carbon atoms that may have a substituent, a cycloalkyl group having 5 to 10 carbon atoms that may have a substituent, a linear or branched alkenyl group having 2 to 6 carbon atoms that may have a substituent, a linear or branched alkyloxy group having 1 to 6 carbon atoms that may have a substituent, a cycloalkyloxy group having 5 to 10 carbon atoms that may have a substituent, a substituted or unsubstituted aryloxy group, a substituted or unsubstituted aromatic hydrocarbon group, a substituted or unsubstituted aromatic heterocyclic group, or a substituted or unsubstituted condensed polycyclic aromatic group, and these groups may be bonded to the aromatic ring which bonded to, to form a ring through a single bond, a substituted or unsubstituted methylene group, an oxygen atom, or a sulfur atom, in which R₁₀ and R₁₁ or R₁₂ to R₁₅ may be bonded to each other to form a ring through a single bond, a substituted or unsubstituted methylene group, an oxygen atom, or a sulfur atom; and Z represents an oxygen atom, a sulfur atom, or a nitrogen atom, provided that in the case where Z represents an oxygen atom or a sulfur atom, Z does not have R₉.
(2) The organic EL device according to the item (1), wherein the amine compound having a benzazole ring structure is represented by the following general formula (1a): 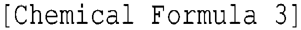 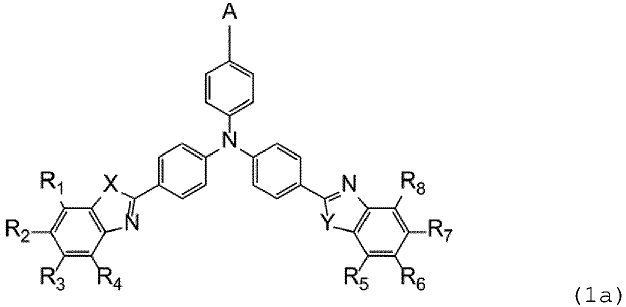 wherein R₁ to R₈, X, Y, and A each are as defined in the general formula (1).
(3) The organic EL device according to the item (1) or (2), wherein in the general formula (1) or the general formula (1a), all R₁ to R₈ represent hydrogen atoms.
(4) The organic EL device according to any one of the items (1) to (3), wherein the capping layer has an extinction coefficient of 0.40 or more at a wavelength in a range of 400 nm or more and 410 nm or less.
(5) The organic EL device according to the item (1) or (2), wherein in the general formula (1) or the general formula (1a), X and Y represent oxygen atoms.
(6) The organic EL device according to the item (1) or (2), wherein in the general formula (1) or the general formula (1a), X and Y represent sulfur atoms.
(7) The organic EL device according to the item (1) or (2), wherein in the general formula (2), Z represents an oxygen atom.
(8) The organic EL device according to the item (1) or (2), wherein in the general formula (2), Z represents a sulfur atom.
(9) The organic EL device according to the item (1) or (2), wherein in the general formula (2), Z represents a nitrogen atom.
(10) The organic EL device according to the item (1) or (2), wherein in the general formula (2), R₉ represents a linking group as a bonding site.
(11) The organic EL device according to the item (1) or (2), wherein in the general formula (2), R₁₀ represents a linking group as a bonding site.
(12) The organic EL device according to the item (1) or (2), wherein in the general formula (2), R₁₃ represents a linking group as a bonding site.
(13) The organic EL device according to the item (9), wherein in the general formula (2), R₉ represents a linking group as a bonding site.
(14) A method for using an amine compound having a benzazole ring structure represented by the general formula (1) or the general formula (1a) having a refractive index of 2.00 or more while light having a wavelength in a range of 400 nm or more and 500 nm or less and a refractive index of 1.90 or more while light having a wavelength in a range of more than 500 nm and 570 nm or less, in a capping layer of an organic EL device.
(15) A method for producing the organic EL device according to any one of the items (1) to (4), the method including forming the capping layer of the organic EL device using an amine compound having a benzazole ring structure represented by the following general formula (1) or the following general formula (1a): 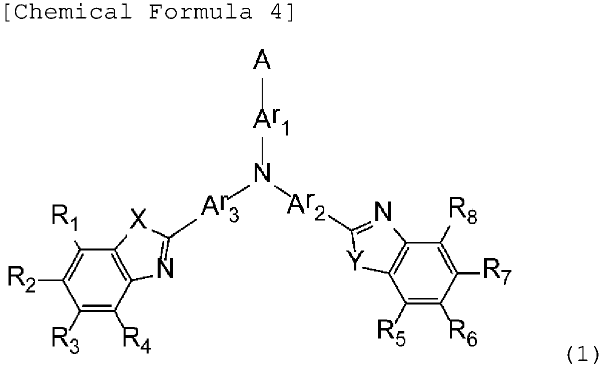
   wherein R₁ to R₈ may be the same or different, each represent a hydrogen atom, a deuterium atom, a fluorine atom, a chlorine atom, a cyano group, a nitro group, a linear or branched alkyl group having 1 to 6 carbon atoms that may have a substituent, a cycloalkyl group having 5 to 10 carbon atoms that may have a substituent, a linear or branched alkenyl group having 2 to 6 carbon atoms that may have a substituent, a linear or branched alkyloxy group having 1 to 6 carbon atoms that may have a substituent, a cycloalkyloxy group having 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or a substituted or unsubstituted aryloxy group, and these groups may be bonded to the benzene ring which bonded to, to form a ring through a single bond, a substituted or unsubstituted methylene group, an oxygen atom, or a sulfur atom, in which R₁ to R₄ or R₅ to R₈ bonded to the same benzene ring may be bonded to each other to form a ring through a single bond, a substituted or unsubstituted methylene group, an oxygen atom, or a sulfur atom; X and Y may be the same or different, and each represent an oxygen atom or a sulfur atom; Ar₁ to Ar₃ may be the same or different, and each represent a divalent group of a substituted or unsubstituted aromatic hydrocarbon, a divalent group of a substituted or unsubstituted aromatic heterocyclic ring, or a divalent group of a substituted or unsubstituted condensed polycyclic aromatic; and A represents a monovalent group represented by the following general formula (2) having a bonding site at any one of R₉ to R₁₅: 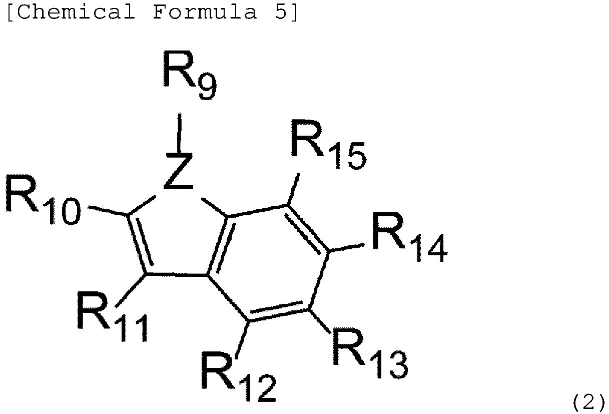
   wherein R₉ to R₁₅ may be the same or different, and each represent a linking group as a bonding site, a hydrogen atom, a deuterium atom, a fluorine atom, a chlorine atom, a cyano group, a nitro group, a linear or branched alkyl group having 1 to 6 carbon atoms that may have a substituent, a cycloalkyl group having 5 to 10 carom atoms that may have a substituent, a linear or branched alkenyl group having 2 to 6 carbon atoms that may have a substituent, a linear or branched alkyloxy group having 1 to 6 carbon atoms that may have a substituent, a cycloalkyloxy group having 5 to 10 carbon atoms that may have a substituent, a substituted or unsubstituted aryloxy group, a substituted or unsubstituted aromatic hydrocarbon group, a substituted or unsubstituted aromatic heterocyclic group, or a substituted or unsubstituted condensed polycyclic aromatic group, and these groups may be bonded to the aromatic ring which bonded to, to form a ring through a single bond, a substituted or unsubstituted methylene group, an oxygen atom, or a sulfur atom, in which R₁₀ and R₁₁ or R₁₂ to R₁₅ may be bonded to each other to form a ring through a single bond, a substituted or unsubstituted methylene group, an oxygen atom, or a sulfur atom; and Z represents an oxygen atom, a sulfur atom, or a nitrogen atom, provided that in the case where Z represents an oxygen atom or a sulfur atom, Z does not have R₉, 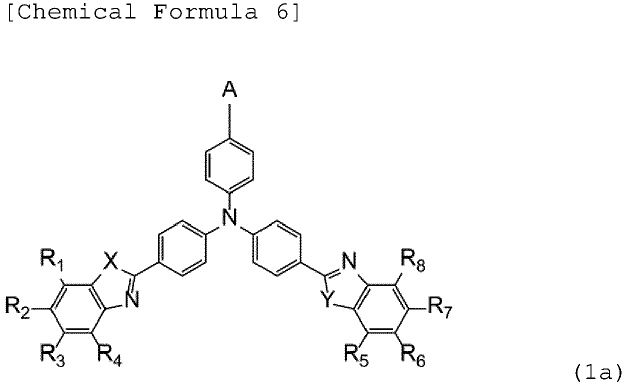
   wherein R₁ to R₈, X, Y, and A each are as defined in the general formula (1).

The "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by R₁ to R₁₅ in the general formula (1), the general formula (2), or the general formula (1a) may be specifically selected from a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzoimidazolyl group, a pyrazolyl group, a dibenzofuranyl group, a dibenzothienyl group, a naphthyridinyl group, a phenanthrolinyl group, an acridinyl group, and a carbolinyl group, and may also be selected from an aryl group having 6 to 30 carbon atoms and a heteroaryl group having 2 to 20 carbon atoms.

In the case where more than one of these groups are bonded to the same aromatic ring (e.g., the benzene ring or the heterocyclic five-membered ring), the groups may be bonded to each other to form a ring through a single bond, a substituted or unsubstituted methylene group, an oxygen atom, or a sulfur atom, or may be bonded to form a ring to the aromatic ring (e.g., the benzene ring or the heterocyclic five-membered ring) bonded to the groups, through a single bond, a substituted or unsubstituted methylene group, an oxygen atom, or a sulfur atom.

Specific examples of the "linear or branched alkyl group of 1 to 6 carbon atoms", the "cycloalkyl group of 5 to 10 carbon atoms", the "linear or branched alkenyl group of 2 to 6 carbon atoms", the "linear or branched alkyloxy group of 1 to 6 carbon atoms", the "cycloalkyloxy group of 5 to 10 carbon atoms" or the "aryloxy group" in the "linear or branched alkyl group of 1 to 6 carbon atoms that may have a substituent", the "cycloalkyl group of 5 to 10 carbon atoms that may have a substituent", the "linear or branched alkenyl group of 2 to 6 carbon atoms that may have a substituent", the "linear or branched alkyloxy group of 1 to 6 carbon atoms that may have a substituent", the "cycloalkyloxy group of 5 to 10 carbon atoms that may have a substituent" or the "substituted or unsubstituted aryloxy group" represented by R₁ to R₁₅ in the general formula (1), the general formula (2), or the general formula (1a) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, a 2-butenyl group, a methyloxy group, an ethyloxy group, an n-propyloxy group, cyclopentyloxy group, cyclohexyloxy group, 1-adamantyloxy group, phenyloxy group, tolyloxy group, and biphenyloxy group.

In the case where more than one of these groups are bonded to the same aromatic ring (e.g., the benzene ring or the heterocyclic five-membered ring), the groups may be bonded to each other to form a ring through a single bond, a substituted or unsubstituted methylene group, an oxygen atom, or a sulfur atom, or may be bonded to form a ring to the aromatic ring (e.g., the benzene ring or the heterocyclic five-membered ring) bonded to the groups, through a single bond, a substituted or unsubstituted methylene group, an oxygen atom or a sulfur atom.

Specific examples of the "substituent" in the "substituted aromatic hydrocarbon group", the "substituted aromatic heterocyclic group", the "substituted condensed polycyclic aromatic group", the "linear or branched alkyl group of 1 to 6 carbon atoms having a substituent", the "cycloalkyl group of 5 to 10 carbon atoms having a substituent", the "linear or branched alkenyl group of 2 to 6 carbon atoms having a substituent", the "linear or branched alkyloxy group of 1 to 6 carbon atoms having a substituent", the "cycloalkyloxy group of 5 to 10 carbon atoms having a substituent" or the "substituted aryloxy group" represented by R₁ to R₁₅ in the general formula (1), the general formula (2), or the general formula (1a) include a deuterium atom; a cyano group; a nitro group; a halogen atom, such as a fluorine atom, a chlorine atom, a bromine atom, and an iodine atom; a silyl group, such as a trimethylsilyl group and a triphenylsilyl group; a linear or branched alkyl group having 1 to 6 carbon atoms, such as a methyl group, an ethyl group, and a propyl group; a linear or branched alkyloxy group having 1 to 6 carbon atoms, such as a methyloxy group, an ethyloxy group, and a propyloxy group; an alkenyl group, such as a vinyl group and an allyl group; an aryloxy group, such as a phenyloxy group and a tolyloxy group; an arylalkyloxy group, such as a benzyloxy group and a phenethyloxy group; an aromatic hydrocarbon group or a condensed polycyclic aromatic group, such as a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, and a triphenylenyl group; and a pyridyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzoimidazolyl group, a pyrazolyl group, a dibenzofuranyl group, a dibenzothienyl group, and a carbolinyl group; and also include an aryl group having 6 to 30 carbon atoms and a heteroaryl group having 2 to 20 carbon atoms. These substituents may be further substituted with the exemplified substituents above.

These substituents may be bonded to each other to form a ring through a single bond, a substituted or unsubstituted methylene group, an oxygen atom, or a sulfur atom.

Specific examples of the "aromatic hydrocarbon", the "aromatic heterocyclic ring", or the "condensed polycyclic aromatics" of the "substituted or unsubstituted aromatic hydrocarbon", the "substituted or unsubstituted aromatic heterocyclic ring", or the "substituted or unsubstituted condensed polycyclic aromatics" in the "divalent group of a substituted or unsubstituted aromatic hydrocarbon", the "divalent group of a substituted or unsubstituted aromatic heterocyclic ring", or the "divalent group of substituted or unsubstituted condensed polycyclic aromatics" represented by Ar₁ to Ar₃ in the general formula (1) include benzene, biphenyl, terphenyl, tetrakisphenyl, styrene, naphthalene, anthracene, acenaphthalene, fluorene, phenanthrene, indane, pyrene, triphenylene, pyridine, pyrimidine, triazine, pyrrole, furan, thiophene, quinoline, isoquinoline, benzofuran, benzothiophene, indoline, carbazole, carboline, benzoxazole, benzothiazole, quinoxaline, benzoimidazole, pyrazole, dibenzofuran, dibenzothiophene, naphthyridine, phenanthroline, and acridine.

The "divalent group of a substituted or unsubstituted aromatic hydrocarbon", the "divalent group of a substituted or unsubstituted aromatic heterocyclic ring", or the "divalent group of substituted or unsubstituted condensed polycyclic aromatics" represented by Ar₁ to Ar₃ in the general formula (1) is a divalent group that results from the removal of two hydrogen atoms from the "aromatic hydrocarbon", the "aromatic heterocyclic ring", or the "condensed polycyclic aromatics" above.

These divalent groups may have a substituents, and examples of the substituent include the same substituents exemplified as the "substituent" of the "substituted aromatic hydrocarbons group", the "substituted aromatic heterocyclic group" the "substituted condensed polycyclic aromatic group", the "linear or branched alkyl group of 1 to 6 carbon atoms having a substituent", the "cycloalkyl group of 5 to 10 carbon atoms having a substituent", the "linear or branched alkenyl group of 2 to 6 carbon atoms having a substituent", the "linear or branched alkyloxy group of 1 to 6 carbon atoms having a substituent", the "cycloalkyloxy group of 5 to 10 carbon atoms having a substituent", or the "substituted aryloxy group" represented by R₁ to R₁₅ in the general formula (1), the general formula (2), or the general formula (1a). These substituents may be bonded to each other to form a ring through a single bond, a substituted or unsubstituted methylene group, an oxygen atom, or a sulfur atom.

In the general formula (1), the general formula (2), or the general formula (1a), X, Y, and Z may be the same or different, and each represent an oxygen atom, a sulfur atom, or a nitrogen atom, provided that in the case where Z represents an oxygen atom or a sulfur atom, Z does not have R₉. X and Y each preferably represent an oxygen atom or a sulfur atom, and both X and Y more preferably represent oxygen atoms or sulfur atoms.

In the general formula (2), any one of R₉ to R₁₅ represents a linking group as a bonding site, and R₉ preferably represents a linking group as a bonding site, a linear or branched alkyl group having 1 to 6 carbon atoms that may have a substituent, a cycloalkyl group having 5 to 10 carom atoms that may have a substituent, a linear or branched alkenyl group having 2 to 6 carbon atoms that may have a substituent, a substituted or unsubstituted aromatic hydrocarbon group, a substituted or unsubstituted aromatic heterocyclic group, or a substituted or unsubstituted condensed polycyclic aromatic group, more preferably a linking group as a bonding site, a substituted or unsubstituted aromatic hydrocarbon group, a substituted or unsubstituted aromatic heterocyclic group, or a substituted or unsubstituted condensed polycyclic aromatic group, and further preferably a linking group as a bonding site or a substituted or unsubstituted aromatic hydrocarbon group.

In the general formula (1), Ar₁ to Ar₃ each preferably represent a divalent group of a substituted or unsubstituted aromatic hydrocarbon, and more preferably a divalent group that results from the removal of two hydrogen atoms from substituted or unsubstituted benzene (i.e., a phenylene group). All Ar₁, Ar₂, and Ar₃ further preferably represent divalent groups that each result from the removal of two hydrogen atoms from substituted or unsubstituted benzene (i.e., phenylene groups).

In the organic EL device of the present invention, the thickness of the capping layer is preferably in a range of 30 nm to 120 nm, and more preferably in a range of 40 nm to 80 nm.

In the organic EL device of the present invention, the refractive index of the capping layer to light transmitted through the capping layer having a wavelength in a range of more than 500 nm and 570 nm or less is preferably 1.90 or more, more preferably 1.95 or more, and further preferably 2.00 or more. The refractive index of the capping layer to light transmitted through the capping layer having a wavelength in a range of 400 nm or more and 500 nm or less is preferably 2.00 or more, and more preferably 2.05 or more.

In the organic EL device of the present invention, the extinction coefficient of the capping layer to light transmitted through the capping layer having a wavelength in a range of 400 nm or more and 410 nm or less is preferably 0.40 or more, and more preferably 0.50 or more.

In the organic EL device of the present invention, the capping layer may be produced as a laminated layer or a mixed layer of two or more kinds of different constitutional materials.

### Advantageous Effects of Invention

The organic EL device of the present invention uses a material that has a high light absorption coefficient to light having a wavelength in a range of 400 nm to 410 nm, and a high refractive index, and is excellent in the stability and durability of the thin film, and light resistance, as the material of the capping layer, and therefore can largely enhance the light extraction efficiency as compared to the ordinary organic EL devices, by providing the capping layer having a higher refractive index than the transparent or translucent electrode, provided outside the transparent or translucent electrode. Furthermore, the capping layer formed with the amine compound having a benzazole ring structure well absorbs light having a wavelength of 400 nm to 410 nm of sunlight, and therefore an organic EL device that has a high efficiency and a long lifetime can be achieved since the inside of the organic EL device is not affected by sunlight.

### Brief Description of Drawings

Fig. 1 is a figure showing structures of compounds (1-1) to (1-12) as an amine compound having a benzazole ring structure represented by a general formula (1) of the present invention.
Fig. 2 is a figure showing structures of compounds (1-13) to (1-21) as the amine compound having a benzazole ring structure represented by the general formula (1) of the present invention.
Fig. 3 is a figure showing structures of compounds (1-22) to (1-24) as the amine compound having a benzazole ring structure represented by the general formula (1) of the present invention.
Fig. 4 is a diagram illustrating configuration of organic EL devices of Examples 10 to 15 and Comparative Examples 1 to 4.

### Description of Embodiments

The benzazole derivative as the major skeleton of the amine compound having a benzazole ring structure represented by the general formula (1) or (1a) of the invention can be synthesized, for example, by the known methods (see, for example, NPL 4). Furthermore, the amine compound having a benzazole ring structure represented by the general formula (1) or (1a) of the invention can be synthesized by conducting coupling reaction between the synthesized halogenated benzazole derivative and an arylamine using a copper catalyst or a palladium catalyst.

The amine compound having a benzazole ring structure represented by the general formula (1) or (1a) of the invention can also be synthesized by derivatizing the halogenated benzazole derivative into a boronic acid derivative or a boronic acid ester derivative, followed by coupling reaction with a halogenated arylamine (see, for example, NPL 5 and NPL 6).

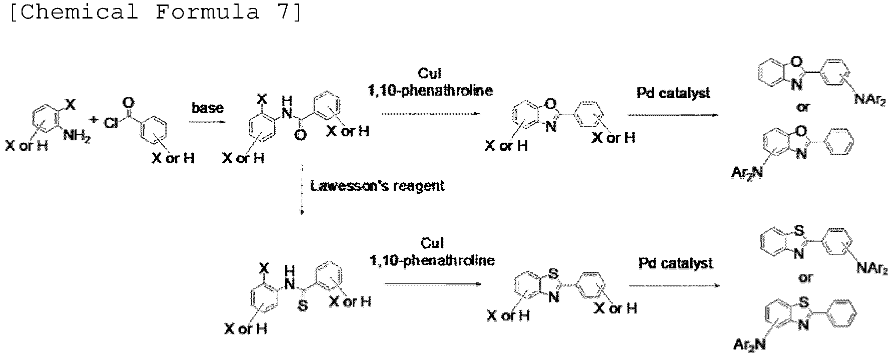

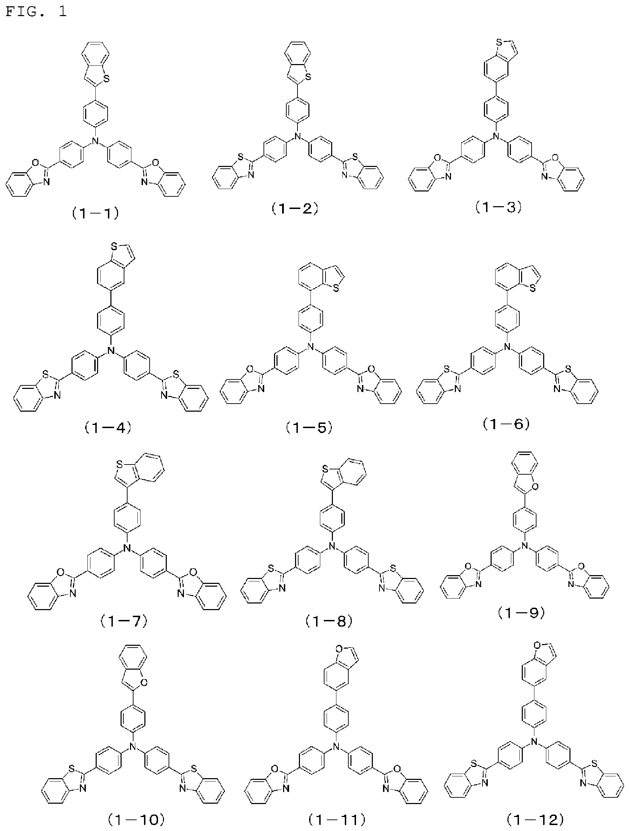

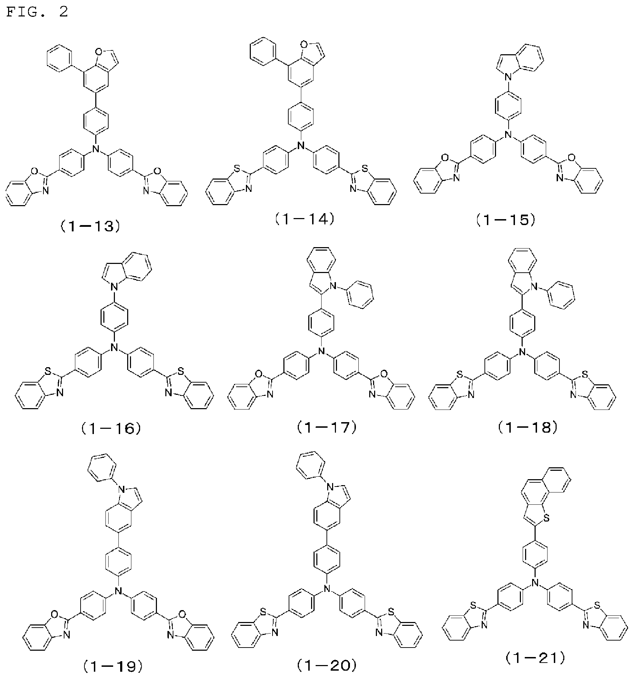

The specific examples of preferred compounds of the amine compound having a benzazole ring structure represented by the general formula (1) or (1a) preferably used in the organic EL devices of the present invention are shown in Figs 1 to 3. The present invention, however, is not restricted to these compounds.

The amine compound having a benzazole ring structure represented by the general formula (1) or (1a), which are suitably used for the organic EL devices of the present invention, is purified by column chromatography, adsorption purification using, for example, a silica gel, activated carbon, or activated white clay, recrystallization or crystallization using a solvent, and finally purification by the sublimation purification method. The compound is identified by NMR analysis. The melting point, the glass transition point (Tg), the refractive index, the extinction coefficient, and the absorbance are measured as material property values. The melting point can be used as an index of the deposition properties, the glass transition point (Tg) can be used as an index of the stability in a thin film state, and the refractive index can be used as an index of the enhancement of the light extraction efficiency. The extinction coefficient can be used as an index of the light resistance. The absorbance can be used as an index of the light absorption capability.

The melting point and the glass transition point (Tg) are measured by using a powder specimen with a high sensitivity differential scanning calorimeter (DSC 3100SA, produced by Bruker AXS).

The refractive index and the extinction coefficient are measured in such a manner that a thin film of 80 nm is formed on a silicon substrate and measured with a spectrometer (F10-RT-UV produced by Filmetrics).

The absorbance is measured in a toluene solvent at a prepared concentration of 10⁻⁵ mol/L, and the absorption coefficient is measured in a toluene solution at prepared four concentrations of 5.0 × 10⁻⁶ mol/L, 1.0 × 10⁻⁵ mol/L, 1.5 × 10⁻⁵ mol/L, and 2.0 × 10⁻⁵ mol/L, with a UV-visible-near-infrared spectrophotometer (V-650, produced by JASCO Corporation).

The organic EL device of the present invention, for example, in the case of a top emission light emitting device, may have a structure including an anode, a hole transport layer, a light emitting layer, an electron transport layer, a cathode, and a capping layer successively formed on a glass substrate, optionally with a hole injection layer between the anode and hole transport layer, an electron blocking layer between the hole transport layer and the light emitting layer, a hole blocking layer between the light emitting layer and an electron transport layer, and an electron injection layer between the electron transport layer and the cathode. Some of the organic layers in the multilayer structure may be omitted or may serve more than one function. For example, a single organic layer may serve as a hole injection layer and a hole transport layer, a hole transport layer and an electron blocking layer, a hole blocking layer and an electron transport layer, or an electron transport layer and an electron injection layer, and so on. Further, any of the layers may be configured to laminate two or more organic layers having the same function, and the hole transport layer may have a two-layer laminated structure, the light emitting layer may have a two-layer laminated structure, the electron transport layer may have a two-layer laminated structure, the capping layer may have a two-layer laminated structure, and so on.

The total thickness of each layer of the organic EL device is preferably 200 nm to 750 nm, more preferably 350 nm to 600 nm. The absorbance is increased by increasing the thickness of the capping layer, and is not particularly limited as far as the reduction of the thickness of the organic EL device is not hampered, and for example, the thickness of the capping layer is preferably 30 nm to 120 nm, and more preferably 40 nm to 80 nm. In this case, good light extraction efficiency can be obtained. The thickness of the capping layer can be changed according to the type of light-emitting material used in the light-emitting device, the thicknesses of the layers of the organic EL device other than the capping layer, and other factors.

An electrode material with a large work function, such as ITO and gold, may be used as the anode of the organic EL device of the present invention.

The hole injection layer of the organic EL device of the present invention is preferably an arylamine compound having a structure in which two or more triphenylamine structures are joined within a molecule via a single bond or a divalent group that does not contain a heteroatom, for example, an arylamine compound having a structure in which two triphenylamine structures are joined within a molecule via a single bond or a divalent group that does not contain a heteroatom, such as a benzidine derivative, a starburst-type triphenylamine derivative, and various triphenylamine tetramers. A porphyrin compound, such as copper phthalocyanine; an accepting heterocyclic compound, such as hexacyanoazatriphenylene; and a coating-type polymer material may also be used. These materials may be individually deposited for film forming, may be used as a single layer deposited mixed with another material, or may be formed as a laminate of individually deposited layers, a laminate of mixedly deposited layers, or a laminate of the individually deposited layer and the mixedly deposited layer. These materials may be formed into a thin film by a vapor deposition method or other known methods, such as a spin coating method and an inkjet method.

The hole transport layer of the organic EL device of this invention is preferably a benzidine derivative, such as N,N'-diphenyl-N,N'-di(m-tolyl)benzidine (TPD), N,N'-diphenyl-N,N'-di(α-naphthyl)benzidine (NPD), and N,N,N',N'-tetrabiphenylylbenzidine, and 1,1-bis[4-(di-4-tolylamino)phenyl]cyclohexane (TAPC), and particularly an arylamine compound having a structure in which two triphenylamine structures are joined within a molecule via a single bond or a divalent group that does not contain a heteroatom, such as N,N,N',N'-tetrabiphenylylbenzidine, and an arylamine compound having only one triphenylamine structure within a molecule. It is also preferable to use an arylamine compound having a structure in which three or more triphenylamine structures are joined within a molecule via a single bond or a divalent group that does not contain a heteroatom, such as various triphenylamine trimers or tetramers and the like. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. In addition, a coating-type polymer material, such as poly(3,4-ethylenedioxythiophene) (PEDOT) / poly(styrene sulfonate) (PSS) may be used as the injection and transport layer of the hole. These materials may be formed into a thin film by a vapor deposition method or other known methods such as a spin coating method and an inkjet method.

The material used for the hole injection layer or the hole transport layer is preferably obtained by p-doping a material commonly used for these layers with trisbromophenylaminehexachloroantimony, a radialene derivative (see, for example, PTL 3), or the like. A polymer compound having, as a part of the compound structure, a structure of a benzidine derivative, such as TPD, may also be used.

The electron-blocking layer of the organic EL device of the present invention may be a compound having an electron blocking effect, including, for example, a carbazole derivative, such as 4,4',4''-tri(N-carbazolyl)triphenylamine (TCTA), 9,9-bis[4-(carbazol-9-yl)phenyl]fluorene, 1,3-bis(carbazol-9-yl)benzene (mCP), and 2,2-bis(4-carbazol-9-yl-phenyl)adamantane (Ad-Cz); and a compound having a triphenylsilyl group and a triarylamine structure, as represented by 9-[4-(carbazol-9-yl)phenyl]-9-[4-(triphenylsilyl)phenyl]-9H-fluorene. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by a vapor deposition method or other known methods such as a spin coating method and an inkjet method.

The light emitting layer of the organic EL device of this invention may be metal complexes of a quinolinol derivative, such as Alq₃, as well as various metal complexes, an anthracene derivative, a bis-styrylbenzene derivative, a pyrene derivative, an oxazole derivative, a polyparaphenylene vinylene derivative, etc. Further, the light emitting layer may be made of a host material and a dopant material. An anthracene derivative is preferably used as the host material, but in addition to the light emitting materials above, a heterocyclic compound having an indole ring as a partial structure of the fused ring, heterocyclic compound having a carbazole ring as a partial structure of fused ring, carbazole derivatives, thiazole derivatives, benzimidazole derivatives, polydialkylfluorene derivatives may be used. Further, as the dopant material, quinacridone, coumarin, rubrene, perylene and their derivatives, a benzopyran derivative, a rhodamine derivative, an amino styryl derivative may be used, and a green light emitting material is particularly preferred. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer.

Further, the light-emitting material may be a phosphorescent material. Phosphorescent materials as metal complexes of metals such as iridium and platinum may be used. Examples of the phosphorescent materials include green phosphorescent materials such as Ir(ppy)₃, blue phosphorescent materials such as FIrpic and FIr6, and red phosphorescent materials such as Btp₂Ir(acac), and green phosphorescent materials are particularly preferred. Here, as the hole injecting and transporting host material, 4,4'-di(N-carbazolyl)biphenyl (CBP), and carbazole derivatives such as TCTA and mCP may be used. As electron transporting host materials, p-bis(triphenylsilyl)benzene (UGH2), 2, 2',2"-(1,3,5-phenylene)-tris(1-phenyl-1H-benzimidazole) (TPBI) may be used. In this way, a high-performance organic EL device can be produced.

To avoid concentration quenching, the doping of the host material with the phosphorescent light-emitting material should preferably be made by co-evaporation in a range of 1 to 30 weight percent with respect to the whole light emitting layer.

Further, Examples of the light-emitting material may be delayed fluorescent-emitting material such as a CDCB derivative of PIC-TRZ, CC2TA, PXZ-TRZ, 4CzIPN or the like (refer to Non-Patent Document 7, for example). These materials may be formed into a thin film by using a vapor deposition method or other known methods such as a spin coating method and an inkjet method.

The hole blocking layer of the organic EL device of the present invention may be formed by using a hole blocking compound, such as phenanthroline derivatives such as bathocuproin (BCP), and the metal complexes of quinolinol derivatives such as aluminum(III) bis(2-methyl-8-quinolinate)-4-phenylphenolate (BAlq), various rare earth complexes, triazole derivatives, triazine derivatives, pyrimidine derivatives, oxadiazole derivatives, and benzazole derivatives. These materials may also serve as the material of the electron transport layer. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by using a vapor deposition method or other known methods such as a spin coating method and an inkjet method.

The electron transport layer of the organic EL device of the present invention may be formed by using various metal complexes, triazole derivatives, triazine derivatives, pyrimidine derivatives, oxadiazole derivatives, pyridine derivatives, benzimidazole derivatives, benzazole derivatives, thiadiazole derivatives, anthracene derivatives, carbodiimide derivatives, quinoxaline derivatives, pyridoindole derivatives, phenanthroline derivatives, and silole derivatives in addition to the metal complexes of quinolinol derivatives such as Alq3 and BAlq. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by using a vapor deposition method or other known methods such as a spin coating method and an inkjet method.

Examples of material used for the electron injection layer of the organic EL device of the present invention can be alkali metal salts such as lithium fluoride and cesium fluoride; alkaline earth metal salts such as magnesium fluoride; metal complexes of quinolinol derivatives such as lithium quinolinol; metal oxides such as aluminum oxide; and metals such as ytterbium (Yb), samarium (Sm), calcium (Ca), strontium (Sr), cesium (Cs). However, the electron injection layer may be omitted in the preferred selection of the electron transport layer and the cathode.

Further, in the electron injection layer or the electron transport layer, a material obtained by further N-doping a material which is commonly used for the layer with a metal such as cesium, or the like can be used.

The cathode of the organic EL device of the present invention may be made of an electrode material with a low work function such as aluminum, an alloy with an even lower work function such as a magnesium-silver alloy, magnesium calcium alloys, a magnesium-indium alloy, or an aluminum-magnesium alloy, or ITO or IZO.

The amine compound having a benzazole ring structure represented by the above general formula (1) or (1a) is preferably used as the capping layer of the organic EL device of the present invention. The compound may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by using a vapor deposition method or other known methods such as a spin coating method and an inkjet method.

The above description refers to an organic EL device with a top emission structure, but the invention is not limited to this, and can also be applied to organic EL devices with bottom emission structures, and organic EL devices with dual emission structures that emit light from both the top and the bottom. In these cases, the electrode in the direction from which light is extracted from the light emitting device to the outside must be transparent or translucent.

The refractive index of the material constituting the capping layer is preferably greater than the refractive index of the adjacent electrode. In other words, the capping layer improves the light extraction efficiency in the organic EL device, but the effect is more effective when the reflectance at the interface between the capping layer and the material in contact with the capping layer is larger, because the effect of light interference is greater. Therefore, the refractive index of the material constituting the capping layer is preferably greater than the refractive index of the adjacent electrode. The refractive index in a range of more than 500 nm and 570 nm or less may be 1.90 or more, more preferably 1.95 or more, and further preferably 2.00 or more. The refractive index in a range of 400 nm or more and 500 nm or less is preferably 2.00 or more, more preferably 2.05 or more, and further preferably 2.10 or more.

The following describes an embodiment of the present invention in more detail based on Examples. The present invention, however, is not restricted to the following Examples as long as they do not go beyond the gist of the invention.

### Example 1

### <Synthesis of Example Compound (1-1)>

4-Benzo[b]thien-2-yl-aniline: 7.4 g, 2-(4-bromophenyl)benzoxazole: 19.8 g, tert-butoxy sodium: 9.5 g, and toluene: 70 mL were added to a reaction vessel, and aerated with nitrogen gas for 30 minutes. tris(dibenzylideneacetone) dipalladium(0): 0.9 g and a 50% (w/v) toluene solution of tri(tert-butyl)phosphine: 0.8 g were added thereto, and the mixture was stirred under heating to reflux overnight. After spontaneously cooling, dispersion washing was carried out at 80°C, the insoluble material was filtered off, and the resulting filtrate was concentrated to obtain a crude product. The crude product was purified by crystallization using a toluene solvent, and the precipitated solid was collected to obtain yellow powder of an example compound (1-1): 7.5 g (yield: 37.3%).

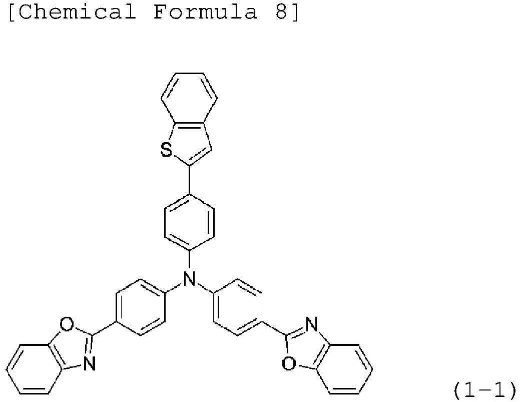

The structure of the obtained yellow powder was identified by NMR.

The ¹H-NMR (CDCl₃) detected 25 hydrogen signals as follows.

δ (ppm) = 8.23-8.19 (4H), 7.87-7.84 (1H), 7.82-7.77 (3H), 7.75-7.71 (2H), 7.62-7.57 (2H), 7.56 (1H), 7.41-7.27 (12H) .

### Example 2

### <Synthesis of Example Compound (1-2)>

4-Benzo[b]thien-2-yl-aniline: 7.4 g, 2-(4-bromophenyl)benzothiazole: 21.0 g, tert-butoxy sodium: 9.5 g, and toluene: 75 mL were added to a reaction vessel, and aerated with nitrogen gas for 30 minutes. tris(dibenzylideneacetone) dipalladium(0): 0.9 g and a 50% (w/v) toluene solution of tri(tert-butyl)phosphine: 0.8 g were added thereto, and the mixture was stirred under heating to reflux overnight. After spontaneously cooling, dispersion washing was carried out at 80°C, the insoluble material was filtered off, and the resulting filtrate was concentrated to obtain a crude product. The crude product was purified by crystallization using a toluene solvent, and the precipitated solid was collected to obtain yellow powder of an example compound (1-2): 3.8 g (yield: 18.0%).

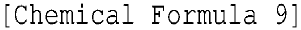

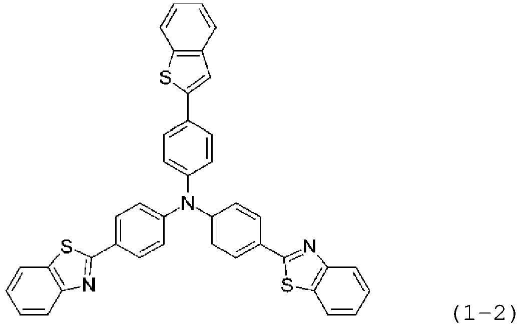

The structure of the obtained yellow powder was identified by NMR.

The ¹H-NMR (CDCl₃) detected 25 hydrogen signals as follows.

δ (ppm) = 8.09-8.04 (6H), 7.94-7.91 (2H), 7.87-7.79 (2H), 7.73-7.70 (2H), 7.53-7.49 (3H), 7.43-7.26 (10H).

### Example 3

### <Synthesis of Example Compound (1-3)>

4-Benzo[b]thien-5-yl-aniline: 5.0 g, 2-(4-bromophenyl)benzoxazole: 13.4 g, tert-butoxy sodium: 6.4 g, and toluene: 50 mL were added to a reaction vessel, and aerated with nitrogen gas for 30 minutes. tris(dibenzylideneacetone) dipalladium(0): 0.6 g and a 50% (w/v) toluene solution of tri(tert-butyl)phosphine: 0.5 g were added thereto, and the mixture was stirred under heating to reflux overnight. After spontaneously cooling, dispersion washing was carried out at 80°C, the insoluble material was filtered off, and the resulting filtrate was concentrated to obtain a crude product. The crude product was purified by crystallization using a toluene-acetone solvent, and the precipitated solid was collected to obtain yellow powder of an example compound (1-3): 8.8 g (yield: 64.9%) .

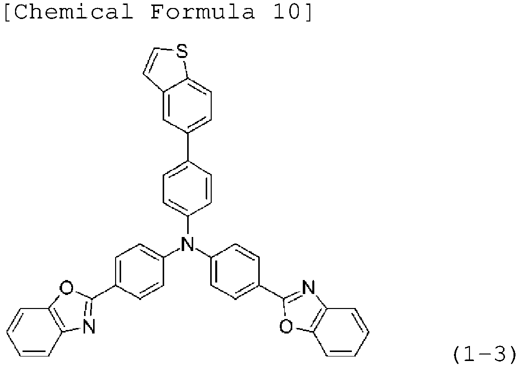

The structure of the obtained yellow powder was identified by NMR.

The ¹H-NMR (CDCl₃) detected 25 hydrogen signals as follows.

δ (ppm) = 8.22-8.19 (4H), 8.08 (1H), 7.99-7.97 (1H), 7.80-7.77 (2H), 7.71-7.58 (5H), 7.53-7.51 (1H), 7.44-7.32 (11H).

### Example 4

### <Synthesis of Example Compound (1-4)>

4-Benzo[b]thien-5-yl-aniline: 5.0 g, 2-(4-bromophenyl)benzothiazole: 14.2 g, tert-butoxy sodium: 6.4 g, and toluene: 50 mL were added to a reaction vessel, and aerated with nitrogen gas for 30 minutes. tris(dibenzylideneacetone) dipalladium(0): 0.6 g and a 50% (w/v) toluene solution of tri(tert-butyl)phosphine: 0.5 g were added thereto, and the mixture was stirred under heating to reflux overnight. After spontaneously cooling, dispersion washing was carried out at 80°C, the insoluble material was filtered off, and the resulting filtrate was concentrated to obtain a crude product. The crude product was purified by crystallization using a toluene-acetone solvent, and the precipitated solid was collected to obtain yellow powder of an example compound (1-4): 8.6 g (yield: 59.83%) .

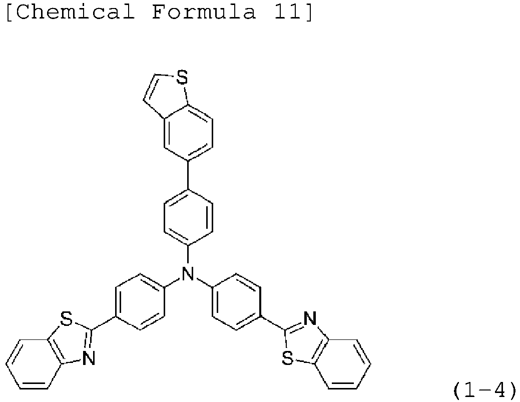

The structure of the obtained yellow powder was identified by NMR.

The ¹H-NMR (CDCl₃) detected 25 hydrogen signals as follows.

δ (ppm) = 8.09-8.02 (7H), 7.99-7.90 (3H), 7.69-7.62 (3H), 7.54-7.49 (3H), 7.46-7.37 (3H), 7.34-7.29 (6H).

### Example 5

### <Synthesis of Example Compound (1-23)>

4-(9H-Carbazol-9-yl)aniline: 6.0 g, 2-(4-bromophenyl)benzoxazole: 14.0 g, tert-butoxy sodium: 6.7 g, and toluene: 60 mL were added to a reaction vessel, and aerated with nitrogen gas for 30 minutes. tris(dibenzylideneacetone) dipalladium(0): 0.6 g and a 50% (w/v) toluene solution of tri(tert-butyl)phosphine: 0.6 g were added thereto, and the mixture was stirred under heating to reflux overnight. After spontaneously cooling, dispersion washing was carried out at 80°C, the insoluble material was filtered off, and the resulting filtrate was concentrated to obtain a crude product. The crude product was purified by crystallization using a monochlorobenzene solvent, and the precipitated solid was collected to obtain yellow powder of an example compound (1-23): 6.7 g (yield: 44.77%) .

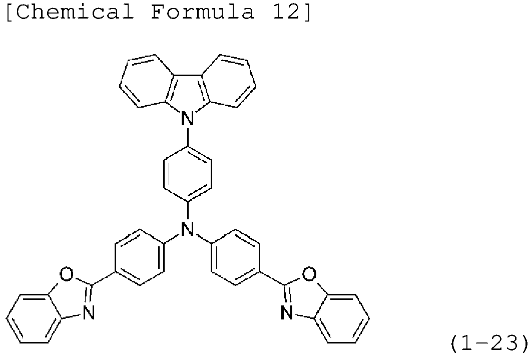

The structure of the obtained yellow powder was identified by NMR.

The ¹H-NMR (CDCl₃) detected 28 hydrogen signals as follows.

δ (ppm) = 8.27-8.17 (6H), 7.81-7.78 (2H), 7.63-7.59 (4H), 7.55-7.31 (16H).

### Example 6

### <Synthesis of Example Compound (1-24)>

4-(9H-Carbazol-9-yl)aniline: 6.0 g, 2-(4-bromophenyl)benzothiazole: 14.8 g, tert-butoxy sodium: 6.7 g, and toluene: 60 mL were added to a reaction vessel, and aerated with nitrogen gas for 30 minutes. tris(dibenzylideneacetone) dipalladium(0): 0.6 g and a 50% (w/v) toluene solution of tri(tert-butyl)phosphine: 0.6 g were added thereto, and the mixture was stirred under heating to reflux overnight. After spontaneously cooling, dispersion washing was carried out at 80°C, the insoluble material was filtered off, and the resulting filtrate was concentrated to obtain a crude product. The crude product was purified by crystallization using a monochlorobenzene solvent, and the precipitated solid was collected to obtain yellow powder of an example compound (1-24): 7.6 g (yield: 48.34%) .

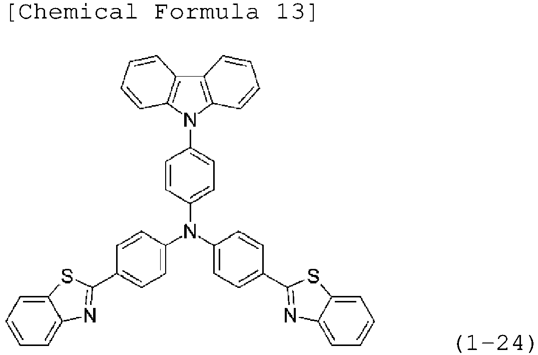

The structure of the obtained yellow powder was identified by NMR.

The ¹H-NMR (CDCl₃) detected 28 hydrogen signals as follows.

δ (ppm) = 8.20-8.18 (2H), 8.12-8.08 (6H), 7.95-7.92 (2H), 7.59-7.32 (18H).

### Example 7

The melting points and the glass transition points (Tg) of the amine compounds having a benzazole ring structure represented by the general formula (1) or (1a) were determined with a high-sensitivity differential scanning calorimeter (DSC3100SA produced by Bruker AXS).

| | Melting point | Glass transition point (Tg) |
|---|---|---|
| Compound (1-1) of Example 1 | 276°C | 120°C |
| Compound (1-2) of Example 2 | 272°C | 118°C |
| Compound (1-3) of Example 3 | 259°C | 116°C |
| Compound (1-4) of Example 4 | 251°C | 113°C |
| Compound (1-23) of Example 5 | 252°C | 132°C |
| Compound (1-24) of Example 6 | 230°C | 130°C |

The results show that the amine compounds having a benzazole ring structure represented by the general formula (1) or (1a) have a glass transition points (Tg) of 100°C or higher, demonstrating that the compounds have a stable thin-film state.

### Example 8

An 80 nm-thick vapor-deposited film was fabricated on a silicon substrate using the amine compound having a benzazole ring structure represented by the general formula (1) or (1a), and the refractive index n at wavelengths of 400 nm, 410 nm, 500 nm, and 570 nm and the extinction coefficient k at wavelengths of 400 nm and 410 nm were measured with a spectrometer (F10-RT-UV produced by Filmetrics). For comparison, comparative compounds (2-1), (2-2), (2-3), and (2-4) of the following structural formulae were also measured (see, for example, PTL 4). The measurement results are summarized in Table 1.

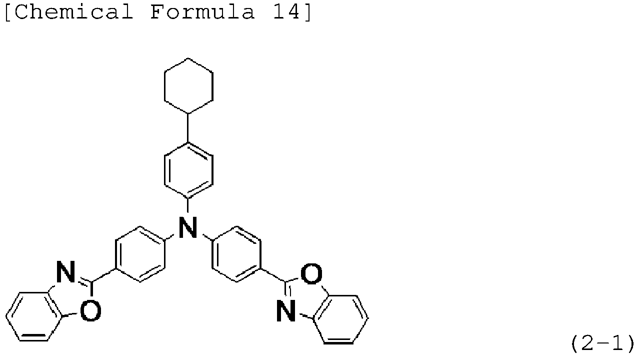

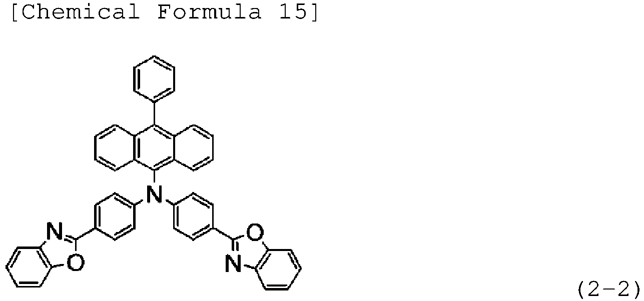

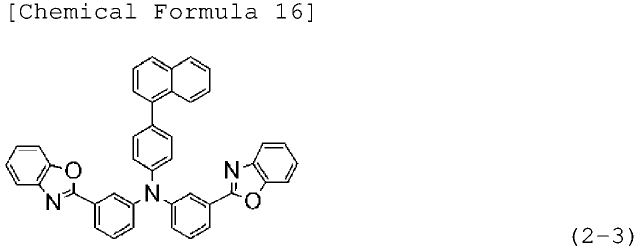

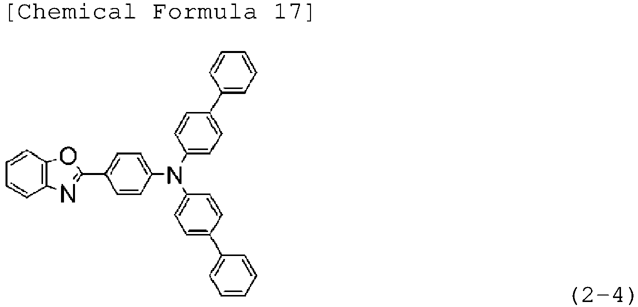

**Table 1**

| | Refractive index n (λ:400nm) | Refractive index n (λ:410nm) | Refractive index n (λ:500nm) | Refractive index n (λ:570nm) | Extinction coefficient k (λ:400nm) | Extinction coefficient k (λ:410nm) |
|---|---|---|---|---|---|---|
| Example compound(1-1) | 2.51 | 2.66 | 2.08 | 1.97 | 0.94 | 0.64 |
| Example compound(1-2) | 2.31 | 2.62 | 2.22 | 2.07 | 1.17 | 1.00 |
| Example compound(1-3) | 2.46 | 2.58 | 2.06 | 1.95 | 0.80 | 0.57 |
| Example compound(1-4) | 2.24 | 2.44 | 2.18 | 2.05 | 0.90 | 0.79 |
| Example compound(1-23) | 2.56 | 2.67 | 2.07 | 1.98 | 0.73 | 0.47 |
| Example compound(1-24) | 2.29 | 2.45 | 2.12 | 2.02 | 0.76 | 0.64 |
| Comparative compound(2-1) | 2.05 | 2.04 | 1.88 | 1.84 | 0.05 | 0.03 |
| Comparative compound(2-2) | 2.35 | 2.27 | 1.94 | 1.88 | 0.14 | 0.06 |
| Comparative compound(2-3) | 2.30 | 2.20 | 1.91 | 1.87 | 0.45 | 0.31 |
| Comparative compound(2-4) | 2.37 | 2.31 | 1.87 | 1.80 | 0.32 | 0.11 |

As shown above, the compounds of the present invention each have a refractive index of 2.00 or more at a wavelength in a range of 400 nm or more and 500 nm or less, and a refractive index of 1.90 or more at a wavelength in a range of more than 500 nm and 570 nm or less, and thus have a refractive index larger than the comparative compounds (2-1), (2-2), (2-3), and (2-4). This indicates that an improvement in light extraction efficiency in the organic EL device can be expected.

Furthermore, the extinction coefficients of the comparative compounds (2-1), (2-2), (2-3) and (2-4) at a wavelength in a range of 400 nm or more and 410 nm or less each are 0.03 to 0.45, while the compounds of the present invention each have a larger value of 0.47 to 1.17. The large extinction coefficients indicates that the capping layer formed with the compound of the present invention well absorbs light at a wavelength of 400 nm to 410 nm of sunlight, not to affect the materials inside the device.

### Example 9

Toluene solutions of the compounds of the present invention were prepared at a concentration of 10⁻⁵ mol/L, and the absorbance thereof at a wavelength of 400 nm and 410 nm was measured with a UV-visible-near-infrared spectrophotometer (V-650 produced by JASCO Corporation). For the absorption coefficient, four different concentrations, 5 × 10⁻⁶ mol/L, 1 × 10⁻⁵ mol/L, 1.5 × 10⁻⁵ mol/L, and 2.0 × 10⁻⁵ mol/L, of toluene solutions were prepared, and measured using a UV-visible-near-infrared spectrophotometer (V-650 produced by JASCO Corporation), and the absorption coefficient was calculated from the calibration curve. For comparison, the comparative compounds (2-1), (2-2), (2-3), and (2-4) of the above structural formulae were also measured. The measurement results are summarized in Table 2.

**Table 2**

| | Peak wavelength (λmax) | Absorbance (λ:400nm) | Absorbance (λ:410nm) | Absorption coefficient |
|---|---|---|---|---|
| Example compound(1-1) | 380nm | 1.05 | 0.37 | 160434 |
| Example compound(1-2) | 387nm | 1.18 | 0.87 | 130663 |
| Example compound(1-3) | 381nm | 1.00 | 0.38 | 130393 |
| Example compound(1-4) | 395nm | 1.38 | 1.18 | 138782 |
| Example compound(1-23) | 382nm | 0.85 | 0.24 | 146599 |
| Example compound(1-24) | 391nm | 1.77 | 1.27 | 185002 |
| Comparative compound(2-1) | 305nm | 0.02 | 0.01 | 114814 |
| Comparative compound(2-2) | 305nm | 0.02 | 0.00 | 98970 |
| Comparative compound(2-3) | 345nm | 0.60 | 0.21 | 100841 |
| Comparative compound(2-4) | 373nm | 0.28 | 0.08 | 88182 |

As shown above, the absorbances at a wavelength of 400 nm of the comparative compounds (2-1), (2-2), (2-3) and (2-4) each are 0.02 to 0.60, while the compounds of the present invention each have a larger value of 0.85 to 1.77. The absorbances at a wavelength of 410 nm of the comparative compounds (2-1), (2-2), (2-3) and (2-4) each are 0.00 to 0.21, while the compounds of the present invention each have a larger value of 0.24 to 1.27. This indicates that light having a wavelength of 400 nm to 410 nm of sunlight is well absorbed. As for the absorption coefficient, the compounds of the present invention each have a value of 130000 or more, and this indicates that the compounds well absorb the light under the same concentration condition, and are materials excellent in light resistance.

### Example 10

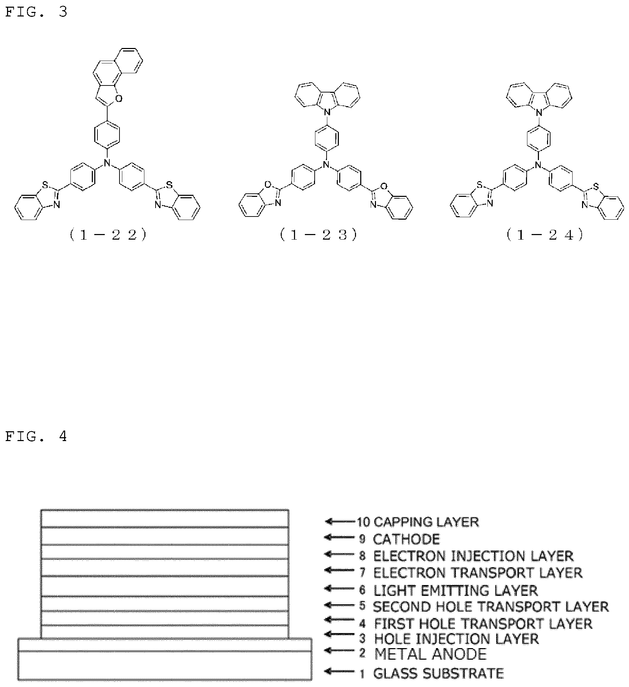

The organic EL device, as shown in Fig. 4, was fabricated by vapor-depositing a hole injection layer 3, a first hole transport layer 4, a second hole transport layer 5, a light emitting layer 6, an electron transport layer 7, an electron injection layer 8, a cathode 9, and a capping layer 10 in this order on a glass substrate 1 on which a reflective ITO electrode as a metal anode 2 had been formed beforehand.

Specifically, as the metal anode 2, an ITO film with a thickness of 50 nm, a silver alloy reflective film with a thickness of 100 nm, and an ITO film with a thickness of 5 nm were deposited on the glass substrate 1 in this order. After ultrasonic cleaning in isopropyl alcohol for 20 minutes, the assembly was dried on a hot plate heated to 250°C for 10 minutes. After UV ozone treatment for 2 minutes, the glass substrate with ITO was installed in a vacuum vapor deposition apparatus, and the pressure was reduced to 0.001 Pa or lower. Subsequently, as the hole injection layer 3 covering the metal anode 2, an electron acceptor (Acceptor-1) of the structural formula below and a compound (3-1) of the structural formula below were formed in a film thickness of 10 nm by dual vapor deposition at a vapor deposition rate ratio Acceptor-1:compound (3-1) = 3: 97 . The first hole transport layer 4 was formed on the hole injection layer 3 by forming the compounds (3-1) of the structural formula below in a film thickness of 70 nm. The second hole transport layer 5 was formed on the first hole transport layer 4 by forming a compound (3-2) of the structural formula below in a film thickness of 10 nm. Then, the light emitting layer 6 was formed on the second hole transport layer 5 in a film thickness of 40 nm by dual vapor deposition of a compound (3-3) of the structural formula below and a compound (3-4) of the structural formula below at a vapor deposition rate ratio of compound (3-3):compound (3-4) = 5:95. The electron transport layer 7 was formed on the light emitting layer 6 in a film thickness of 30 nm by dual vapor deposition of a compound (3-5) of the structural formula below and a compound (3-6) of the structural formula below at a vapor deposition rate ratio of compound (3-5):compound (3-6) = 50:50. The electron injection layer 8 was formed on the electron transport layer 7 by forming lithium fluoride in a film thickness of 1 nm. On top of the electron injection layer 8, a magnesium silver alloy was formed as the cathode 9 with a thickness of 12 nm. Finally, the compound (1-1) of Example 1 was formed as the capping layer 10 with a film thickness of 60 nm. The characteristics of the organic EL device thus fabricated were measured in the atmosphere at an ordinary temperature.

Table 3 summarizes the results of the emission characteristics measurements performed by applying a DC voltage to the fabricated organic EL device.

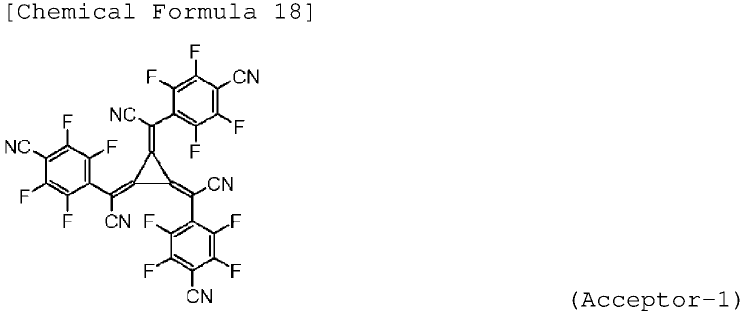

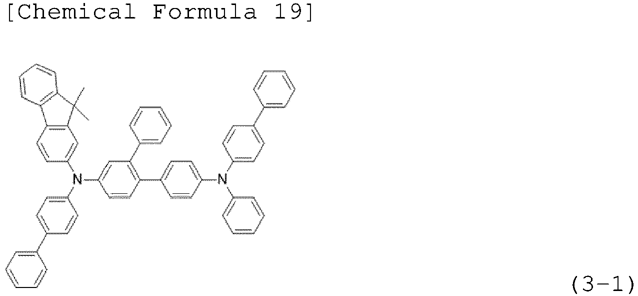

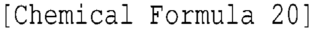

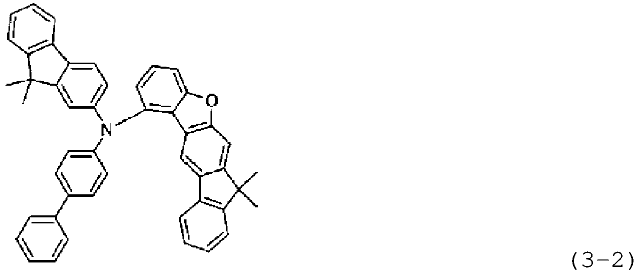

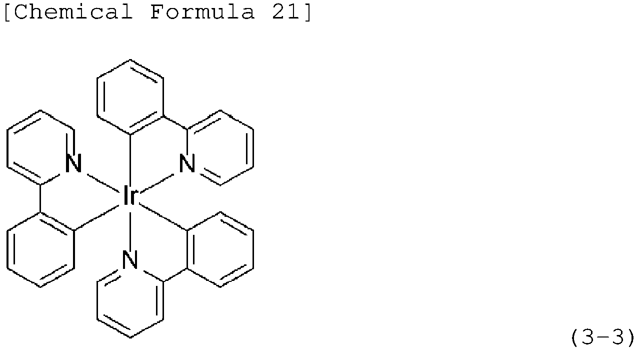

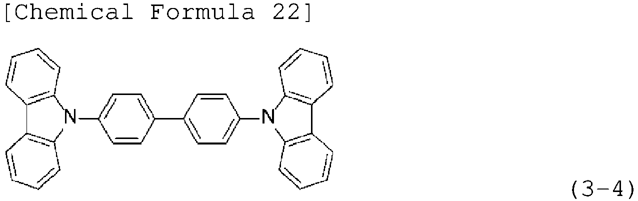

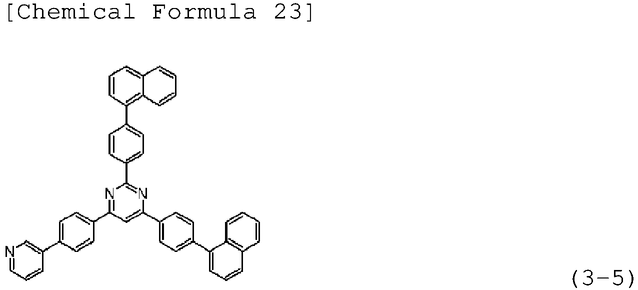

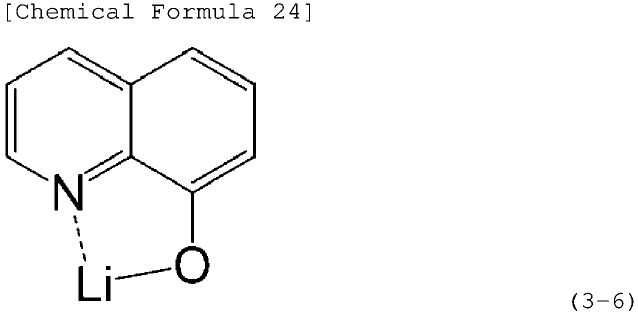

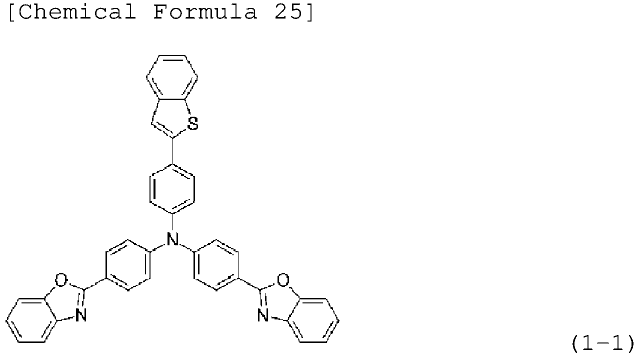

### Example 11

An organic EL device was fabricated under the same conditions as in Example 10 except that the capping layer 10 was formed by using the compound (1-2) of Example 2 instead of the compound (1-1) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

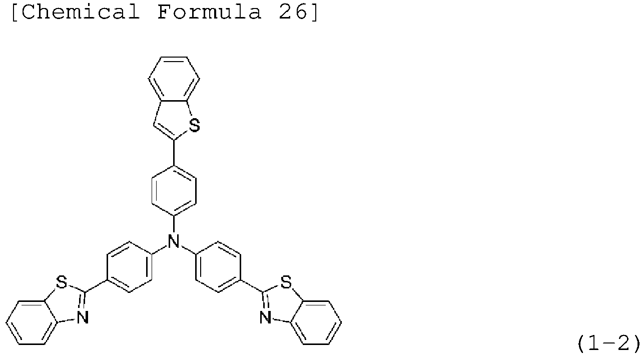

### Example 12

An organic EL device was fabricated under the same conditions as in Example 10 except that the capping layer 10 was formed by using the compound (1-3) of Example 3 instead of the compound (1-1) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

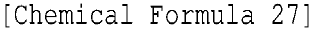

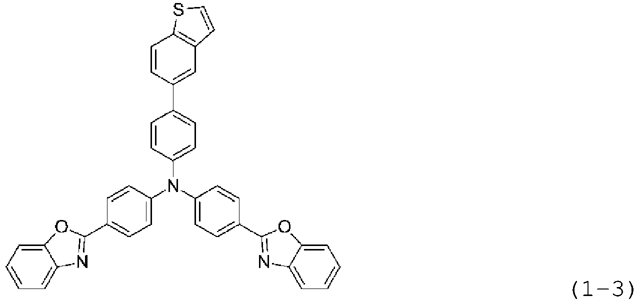

### Example 13

An organic EL device was fabricated under the same conditions as in Example 10 except that the capping layer 10 was formed by using the compound (1-4) of Example 4 instead of the compound (1-1) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

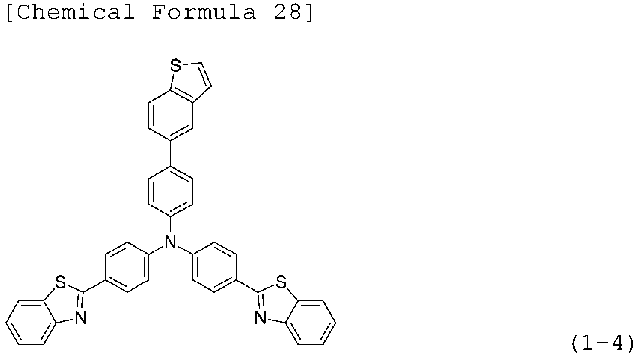

### Example 14

An organic EL device was fabricated under the same conditions as in Example 10 except that the capping layer 10 was formed by using the compound (1-23) of Example 5 instead of the compound (1-1) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

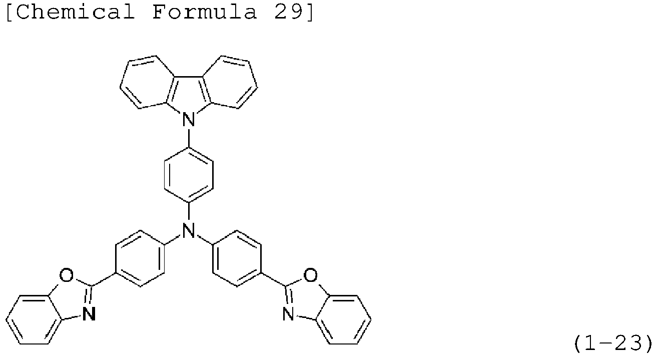

### Example 15

An organic EL device was fabricated under the same conditions as in Example 10 except that the capping layer 10 was formed by using the compound (1-24) of Example 6 instead of the compound (1-1) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

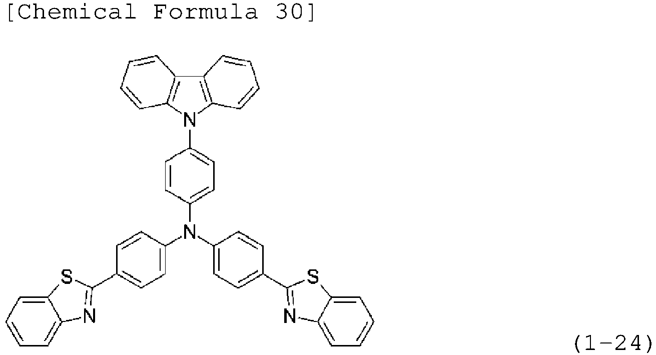

### Comparative Example 1

For comparison, an organic EL device was fabricated under the same conditions as in Example 10 except that the capping layer 10 was formed by using a comparative compound (2-1) of the following structural formula instead of the compound (1-1) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

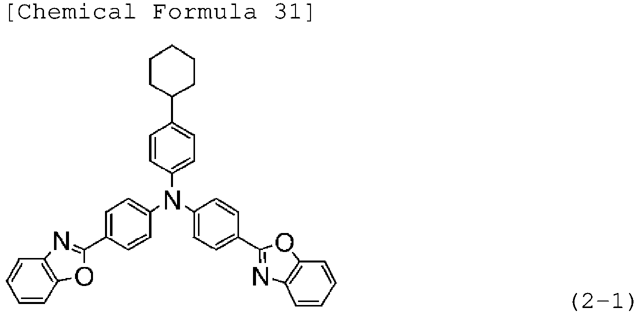

### Comparative Example 2

For comparison, an organic EL device was fabricated under the same conditions as in Example 10 except that the capping layer 10 was formed by using a comparative compound (2-2) of the following structural formula instead of the compound (1-1) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

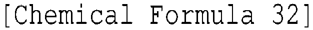

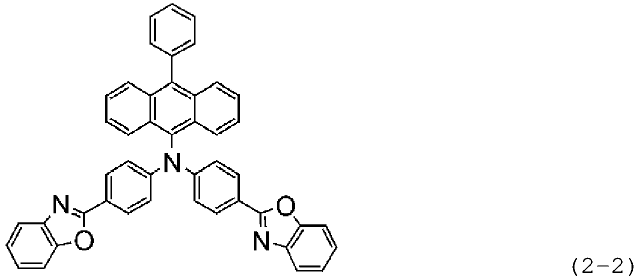

### Comparative Example 3

For comparison, an organic EL device was fabricated under the same conditions as in Example 10 except that the capping layer 10 was formed by using a comparative compound (2-3) of the following structural formula instead of the compound (1-1) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

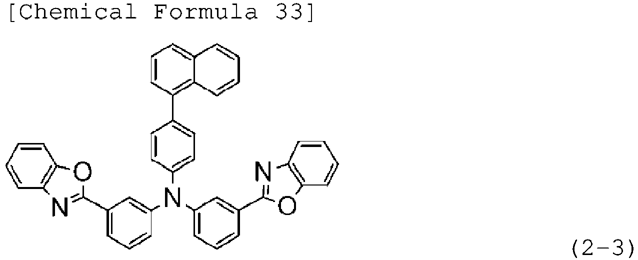

### Comparative Example 4

For comparison, an organic EL device was fabricated under the same conditions as in Example 10 except that the capping layer 10 was formed by using a comparative compound (2-4) of the following structural formula instead of the compound (1-1) of Example 1. The characteristics of the organic EL device thus fabricated were measured in the atmosphere at an ordinary temperature.

Table 3 summarizes the results of the emission characteristics measurements performed by applying a DC voltage to the fabricated organic EL device.

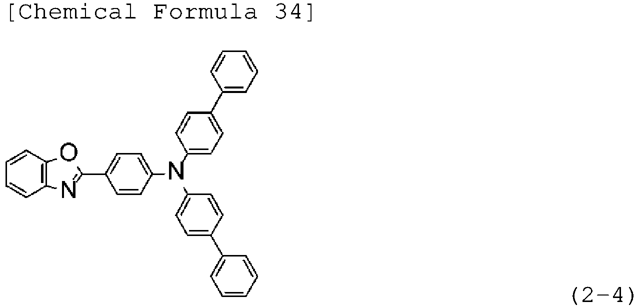

Table 3 summarizes the results of the device lifetime measurements performed with the organic EL devices fabricated in Examples 10 to 15 and Comparative Examples 1 to 4. The device lifetime was measured as the time (attenuation to 95%) taken for the luminance to decay to 95%, with the initial luminance as 100%, when carrying out constant current drive of 10 mA/cm².

**Table 3**

| | Capping layer | Voltage[V] (@10mA/cm2) | Luminance [cd/m2] (@10mA/cm2) | Luminous efficiency[cd/A] (@10mA/cm2) | Power efficiency[lm/W] (@10mA/cm2) | Device lifetime attenuation to 95% |
|---|---|---|---|---|---|---|
| Example 10 | Example compound(1-1) | 3.98 | 16308 | 163.15 | 128.81 | 384hr. |
| Example 11 | Example compound(1-2) | 4.01 | 17414 | 174.19 | 136.53 | 361hr. |
| Example 1 2 | Example compound(1-3) | 4.02 | 16278 | 162.86 | 127.41 | 378hr. |
| Example 13 | Example compound(1-4) | 4.01 | 17087 | 170.94 | 133.80 | 365hr. |
| Example 14 | Example compound(1-23) | 4.01 | 16444 | 164.47 | 128.93 | 373hr. |
| Example! 5 | Example compound(1-24) | 4.02 | 16742 | 167.49 | 131.06 | 358hr. |
| Comparative Example 1 | Comparative compound(2-1) | 4.02 | 13139 | 131.45 | 102.78 | 291hr. |
| Comparative Example 2 | Comparative compound(2-2) | 4.01 | 14625 | 146.30 | 114.55 | 302hr. |
| Comparative Example 3 | Comparative compound(2-3) | 4.06 | 13814 | 138.29 | 107.02 | 301hr. |
| Comparative Example 4 | Comparative compound(2-4) | 4.03 | 13022 | 130.31 | 101.68 | 257hr. |

As shown in Table 3, the driving voltage at a current density of 10 mA/cm² was almost the same for the devices in Comparative Examples 1 to 4 using the comparative compounds and Examples 10 to 15 using the compounds of the present invention. However, the luminance, the luminous efficiency, the power efficiency, and the device lifetime were significantly improved in the devices of Examples 10 to 15 using the compounds of the present invention, as compared to the devices of Comparative Examples 1 to 4 using the comparative compounds. This indicates that the light extraction efficiency can be significantly improved by including a material with a high refractive index, which is suitably used for the organic EL devices of the present invention, in the capping layer.

### Industrial Applicability

As described above, the amine compound having a benzazole ring structure represented by general formula (1) has a high absorption coefficient, a high refractive index, which can significantly improve the light extraction efficiency and the stability in thin film state, and therefore is excellent as a compound used in a capping layer of an organic EL device. With the organic EL device of the present invention fabricated by using the compound, a high efficiency can be obtained, and the durability and the light resistance can be improved so that sunlight is absorbed and does not affect the materials inside the device. The use of this compound, which has no absorption in the blue, green, and red wavelength regions, makes it particularly suitable for displaying clear, bright images with good color purity.

Furthermore, the amine compound having a benzazole ring structure represented by general formula (1) used in the capping layer of the organic EL device of the present invention can form a film at a temperature of 400°C or lower, and therefore can optimize the light extraction efficiency in each of colors by using a high definition mask without damaging the light emitting device, enabling the favorable application to a full-color display. For example, it is now possible to develop applications in home appliances and lighting.

### Reference Sign List

- 1: Glass substrate
- 2: Metal Anode
- 3: Hole injection layer
- 4: First hole transport layer
- 5: Second hole transport layer
- 6: Light emitting layer
- 7: Electron transport layer
- 8: Electron injection layer
- 9: Cathode
- 10: Capping layer

## Claims

1. An organic electroluminescent device in this order comprising at least an anode electrode, a hole transport layer, a light emitting layer, an electron transport layer, a cathode electrode, and a capping layer, the capping layer having a refractive index of 2.00 or more while light transmitted through the capping layer having a wavelength in a range of 400 nm or more and 500 nm or less, and a refractive index of 1.90 or more while light transmitted through the capping layer having a wavelength in a range of more than 500 nm and 570 nm or less, and the organic EL device containing an amine compound having a benzazole ring structure represented by the following general formula (1): 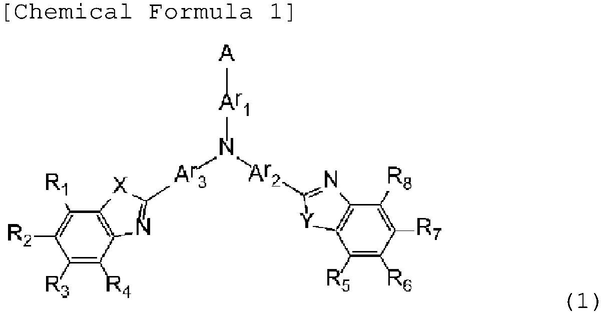
wherein R₁ to R₈ may be the same or different, each represent a hydrogen atom, a deuterium atom, a fluorine atom, a chlorine atom, a cyano group, a nitro group, a linear or branched alkyl group having 1 to 6 carbon atoms that may have a substituent, a cycloalkyl group having 5 to 10 carbon atoms that may have a substituent, a linear or branched alkenyl group having 2 to 6 carbon atoms that may have a substituent, a linear or branched alkyloxy group having 1 to 6 carbon atoms that may have a substituent, a cycloalkyloxy group having 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or a substituted or unsubstituted aryloxy group, and these groups may be bonded to the benzene ring which bonded to, to form a ring through a single bond, a substituted or unsubstituted methylene group, an oxygen atom, or a sulfur atom, in which R₁ to R₄ or R₅ to R₈ bonded to the same benzene ring may be bonded to each other to form a ring through a single bond, a substituted or unsubstituted methylene group, an oxygen atom, or a sulfur atom; X and Y may be the same or different, and each represent an oxygen atom or a sulfur atom; Ar₁ to Ar₃ may be the same or different, and each represent a divalent group of a substituted or unsubstituted aromatic hydrocarbon, a divalent group of a substituted or unsubstituted aromatic heterocyclic ring, or a divalent group of a substituted or unsubstituted condensed polycyclic aromatic; and A represents a monovalent group represented by the following general formula (2) having a bonding site at any one of R₉ to R₁₅: 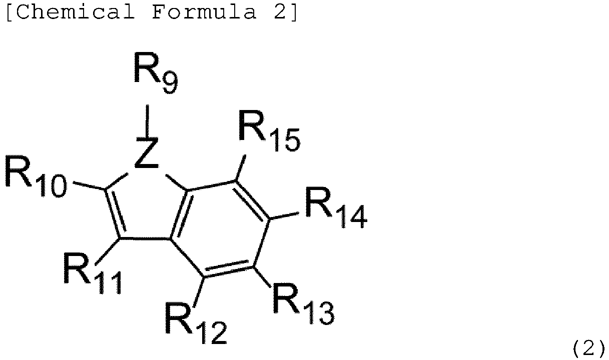
wherein R₉ to R₁₅ may be the same or different, and each represent a linking group as a bonding site, a hydrogen atom, a deuterium atom, a fluorine atom, a chlorine atom, a cyano group, a nitro group, a linear or branched alkyl group having 1 to 6 carbon atoms that may have a substituent, a cycloalkyl group having 5 to 10 carom atoms that may have a substituent, a linear or branched alkenyl group having 2 to 6 carbon atoms that may have a substituent, a linear or branched alkyloxy group having 1 to 6 carbon atoms that may have a substituent, a cycloalkyloxy group having 5 to 10 carbon atoms that may have a substituent, a substituted or unsubstituted aryloxy group, a substituted or unsubstituted aromatic hydrocarbon group, a substituted or unsubstituted aromatic heterocyclic group, or a substituted or unsubstituted condensed polycyclic aromatic group, and these groups may be bonded to the aromatic ring which bonded to, to form a ring through a single bond, a substituted or unsubstituted methylene group, an oxygen atom, or a sulfur atom, in which R₁₀ and R₁₁ or R₁₂ to R₁₅ may be bonded to each other to form a ring through a single bond, a substituted or unsubstituted methylene group, an oxygen atom, or a sulfur atom; and Z represents an oxygen atom, a sulfur atom, or a nitrogen atom, provided that in the case where Z represents an oxygen atom or a sulfur atom, Z does not have R₉.

2. The organic electroluminescent device according to claim 1, wherein the amine compound having a benzazole ring structure is represented by the following general formula (1a): 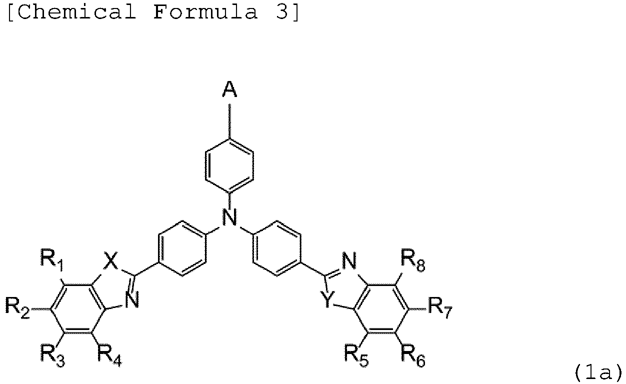 wherein R₁ to R₈, X, Y, and A each are as defined in the general formula (1).

3. The organic electroluminescent device according to claim 1 or 2, wherein in the general formula (1) or the general formula (1a), all R₁ to R₈ represent hydrogen atoms.

4. The organic electroluminescent device according to any one of claims 1 to 3, wherein the capping layer has an extinction coefficient of 0.40 or more at a wavelength in a range of 400 nm or more and 410 nm or less.

5. A method for producing the organic electroluminescent device according to any one of claims 1 to 4, the method comprising forming the capping layer of the organic electroluminescent device using an amine compound having a benzazole ring structure represented by the following general formula (1) or the following general formula (1a): 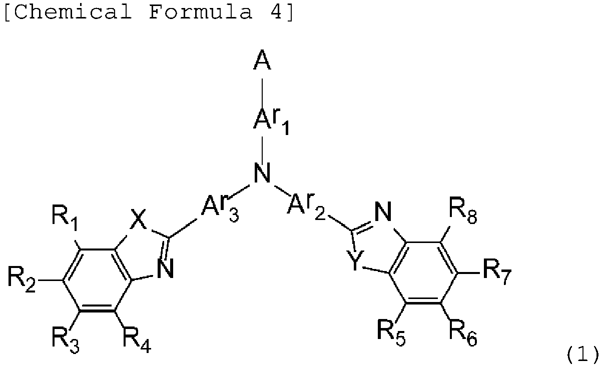
wherein R₁ to R₈ may be the same or different, each represent a hydrogen atom, a deuterium atom, a fluorine atom, a chlorine atom, a cyano group, a nitro group, a linear or branched alkyl group having 1 to 6 carbon atoms that may have a substituent, a cycloalkyl group having 5 to 10 carbon atoms that may have a substituent, a linear or branched alkenyl group having 2 to 6 carbon atoms that may have a substituent, a linear or branched alkyloxy group having 1 to 6 carbon atoms that may have a substituent, a cycloalkyloxy group having 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or a substituted or unsubstituted aryloxy group, and these groups may be bonded to the benzene ring which bonded to, to form a ring through a single bond, a substituted or unsubstituted methylene group, an oxygen atom, or a sulfur atom, in which R₁ to R₄ or R₅ to R₈ bonded to the same benzene ring may be bonded to each other to form a ring through a single bond, a substituted or unsubstituted methylene group, an oxygen atom, or a sulfur atom; X and Y may be the same or different, and each represent an oxygen atom or a sulfur atom; Ar₁ to Ar₃ may be the same or different, and each represent a divalent group of a substituted or unsubstituted aromatic hydrocarbon, a divalent group of a substituted or unsubstituted aromatic heterocyclic ring, or a divalent group of a substituted or unsubstituted condensed polycyclic aromatic; and A represents a monovalent group represented by the following general formula (2) having a bonding site at any one of R₉ to R₁₅: 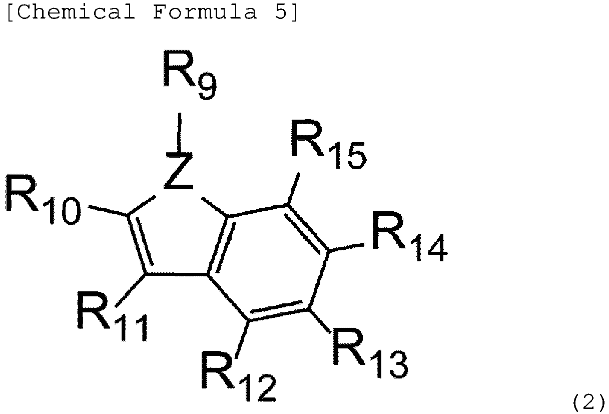
wherein R₉ to R₁₅ may be the same or different, and each represent a linking group as a bonding site, a hydrogen atom, a deuterium atom, a fluorine atom, a chlorine atom, a cyano group, a nitro group, a linear or branched alkyl group having 1 to 6 carbon atoms that may have a substituent, a cycloalkyl group having 5 to 10 carom atoms that may have a substituent, a linear or branched alkenyl group having 2 to 6 carbon atoms that may have a substituent, a linear or branched alkyloxy group having 1 to 6 carbon atoms that may have a substituent, a cycloalkyloxy group having 5 to 10 carbon atoms that may have a substituent, a substituted or unsubstituted aryloxy group, a substituted or unsubstituted aromatic hydrocarbon group, a substituted or unsubstituted aromatic heterocyclic group, or a substituted or unsubstituted condensed polycyclic aromatic group, and these groups may be bonded to the aromatic ring which bonded to, to form a ring through a single bond, a substituted or unsubstituted methylene group, an oxygen atom, or a sulfur atom, in which R₁₀ and R₁₁ or R₁₂ to R₁₅ may be bonded to each other to form a ring through a single bond, a substituted or unsubstituted methylene group, an oxygen atom, or a sulfur atom; and Z represents an oxygen atom, a sulfur atom, or a nitrogen atom, provided that in the case where Z represents an oxygen atom or a sulfur atom, Z does not have R₉, 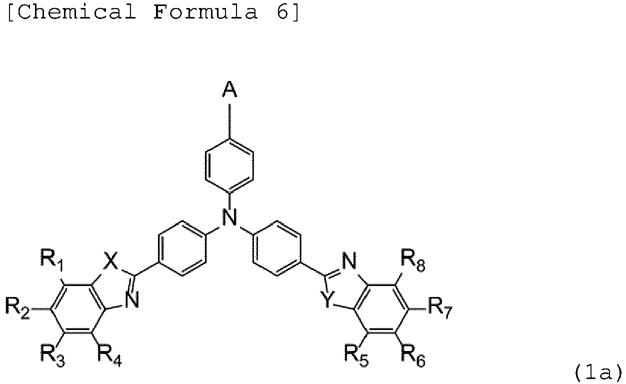
wherein R₁ to R₈, X, Y, and A each are as defined in the general formula (1).
